Europäisches Patentamt

European Patent Office

Office européen des brevets

(19)

(11) Veröffentlichungsnummer: **0 108 882**
**B1**

(12) **EUROPÄISCHE PATENTSCHRIFT**

(45) Veröffentlichungstag der Patentschrift:
22.07.87

(21) Anmeldenummer: 83108864.6

(22) Anmeldetag: 06.09.83

(51) Int. Cl.⁴: **A 61 K 9/22**

(54) **Presslinge mit retardierter Wirkstofffreisetzung und Verfahren zu deren Herstellung.**

(30) Priorität: 15.10.82 AT 3791/82

(43) Veröffentlichungstag der Anmeldung:
23.05.84 Patentblatt 84/21

(45) Bekanntmachung des Hinweises auf die Patenterteilung:
22.07.87 Patentblatt 87/30

(84) Benannte Vertragsstaaten:
AT BE CH DE FR GB IT LI LU NL SE

(56) Entgegenhaltungen:
FR-A-2 070 153
FR-A-2 126 270
FR-A-2 400 950

ADVANCES IN MICROBIOL. PHYSIOLOGY, Band 10, 1973, Seiten 203-260, London, GB, E.A. DAWES u.a.: "Energy reserve polymers in micro-organisms" Teil IV "Poly-beta-hydroxybutyrate"
CHEMICAL ABSTRACTS, Band 99, Nr. 6, 8. August 1983, Seite 314, Nr. 43465s, Columbus, Ohio, US, W. KORSATKO u.a.: "Poly-D(-)-3-hydroxybutyric acid (PHBA) - a biodegradable carrier for long term medication dosage. 1. Development of parenteral matrix tablets for long term application of pharmaceuticals"
J. Vergara, Makromolekulare Chemie 178, 267-270 (1977)

(73) Patentinhaber: CHEMIE LINZ AKTIENGESELLSCHAFT, St. Peter- Strasse 25, A-4020 Linz (AT)

(84) Benannte Vertragsstaaten:
BE CH FR GB IT LI LU NL SE AT

(73) Patentinhaber: Lentia Gesellschaft mit beschränkter Haftung, Arabellastrasse 4 Postfach 81 05 09, D-8000 München 81 (DE)

(84) Benannte Vertragsstaaten: DE

(72) Erfinder: Korsatko, Werner, Dr., Kärntnerstrasse 432, A-8054 Graz (AT)
Erfinder: Wabnegg, Brigitta, Kärntnerstrasse 432, A-8054 Graz (AT)

## Beschreibung

Die vorliegende Erfindung betrifft Preßlinge mit retardierter Wirkstoff-Freisetzung für die orale und parenterale Langzeitapplikation von Arzneistoffen und ein Verfahren zur Herstellung dieser Preßlinge.

Es ist schon seit längerer Zeit bekannt, daß Arzneiformen, welche nach einmaliger Verabreichung einen langanhaltenden, kontinuierlichen Ausstrom des Wirkstoffs gewährleisten, in der klinischen Praxis signifikante Vorteile bieten. Für eine große Anzahl pharmakologisch wichtiger Substanzen sind daher schon galenische Darreichungsformen mit verzögerter Wirkstofffreigabe für die orale und parenterale Applikation (vgl. dazu Remington's Pharmaceutical Sciences, Herausgeber Mack Publishing Company, Easton, Pennsylvania. USA, 1975, Seiten 1618 bis 1643) aber auch für die topische Applikation (beispielsweise GB-PS 1 351 409) entwickelt worden.

Eine bekannte Methode zur Herstellung von Retardpräparaten besteht darin, daß man den Wirkstoff in ein unverdauliches bzw. unlösliches Gerüst (Matrix) einbettet und dann zu Arzneiformen verarbeitet. Als Gerüst- oder Hilfsstoffe werden meistens unverdauliche, säureunlösliche Makromoleküle, beispielsweise Cellulosederivate oder Kunststoffe, wie Polyvinylchlorid, Polyvinylacetat, Polyäthylen oder Polymethacrylat verwendet. Diese Gerüststoffe gewähren zwar eine langanhaltende und kontinuierliche Freisetzung des Wirkstoffes und lassen sich relativ gut verpressen, haben aber den Nachteil, daß sie biologisch nicht abbaubar sind. Dadurch treten einerseits bei peroraler Gabe des Retardpräparates unerwünschte Belastungen des Magen-Darmtrakts auf und andererseits muß beispielsweise bei der Verwendung von Implantaten das nach Freisetzung des Wirkstoffs im Körper zurückbleibende, biologisch nicht abbaubare Gerüst durch einen operativen Eingriff wieder entfernt werden. Es hat daher nicht an Versuchen gefehlt, bei der Herstellung von Depotarzneiformen biologisch abbaubare Polymere als Hilfsstoff bzw. als Arzneistoffträger einzusetzen.

Aus der US-PS 3 773 919 sind Arzneimittelzubereitungen mit verzögerter Wirkstofffreigabe bekannt, bei denen die Matrix aus Polymilchsäure in der racemischen oder optisch aktiven Form, aus Polyglykolsäure oder aus Mischpolymerisaten der beiden Säuren (Polylactide) besteht. Bei diesen Präparaten wird das Trägermaterial nach der Verabreichung unter physiologischen Bedingungen hydrolytisch gespalten und gibt dabei den Wirkstoff frei, sodaß die Matrix nach Beendigung der Wirkstoff-Freisetzung biologisch abgebaut ist. Formulierungen mit Polylactiden zeigen eine gute Retardwirkung von einigen Wochen bis zu einem Jahr, wenn man das Präparat beispielsweise im Körper des Patienten implantiert.

Ein Nachteil bei der Verwendung dieser biologisch abbaubaren Polymeren besteht darin, daß die Arzneiformengebung sehr schwierig durchzuführen und technisch aufwendig ist. Wegen der schlechten Mischbarkeit, der starken elektrostatischen Aufladung und der schlechten Fließeigenschaften sowie hohen Elastizität der Polylactide gelingt es kaum oder nur unter großem technischem Aufwand, wie Arbeiten unter flüssigem Stickstoff, homogene Mischungen mit Arzneistoffen herzustellen und direkt zu verpressen. Präparate, die auf diese Weise hergestellt werden, sind inhomogen und liefern auch keine zufriedenstellenden Ergebnisse in der Wirkstoff-Freisetzung. Die Arzneiformengebung beschränkt sich bei den Polylactiden als Verzögerungszusatz daher hauptsächlich auf die Herstellung von Filmen nach dem Schmelzpreßverfahren oder auf die Herstellung von Mikrokapseln oder Mikrokugeln durch Sprühtrocknung oder Phasentrennung. Für das Schmelzpreßverfahren eignen sich aber nur thermisch stabile Arzneistoffe und die Verwendung von organischen Lösungsmitteln bei der Sprühtrocknung oder Phasentrennung erfordert aus Gründen der Gesundheit und des Umweltschutzes erhebliche technische Schutzvorrichtungen, sodaß beide Verfahren nur ungern angewendet werden.

Schließlich ist in der DE-OS 28 36 044 ein Verfahren zur Herstellung von Mikrokugeln mit einer polymeren Außenschicht durch Phasentrennung beschrieben und es ist dort auch erwähnt, daß das Polymer neben anderen Polymeren auch eine nicht näher definierte Polyhydroxybuttersäure sein kann. Eine nähere Erläuterung wird aber ausschließlich für die Herstellung von Mikrokugeln aus Polymilchsäure gegeben und in der genannten Druckschrift ist kein einziges Beispiel für die Verwendung von Polyhydroxybuttersäure enthalten.

Es wurde nun überraschenderweise gefunden, daß man den Vorteil, den biologisch abbaubare, homogene Matrixformen bieten, mit einfachen Methoden in der Arzneiformengebung verbinden kann, wenn man als polymeres Trägermaterial Poly-D(-)-3-hydroxybuttersäure verwendet.

Gegenstand der vorliegenden Erfindung sind demnach Preßlinge mit retardierter Wirkstoff-Freisetzung zur oralen und parenteralen Applikation von Arzneistoffen, bestehend aus dem Wirkstoff und einem polymeren Trägermaterial, die dadurch gekennzeichnet sind, daß sie wenigstens einen pharmazeutisch aktiven Wirkstoff in homogener Mischung mit mindestens 20 Gew.% Poly-D(-) -3-hydroxybuttersäure, bezogen auf die Wirkstoffmenge, enthalten.

Als Arzneiformen für die erfindungsgemäßen Preßlinge kommen vor allem Tabletten jeder Größe und Form sowie Drageekerne in Frage. Jedoch eignen sich natürlich auch andere Arzneiformen, beispielsweise sehr kleine Komprimate, die anschließend in Kapseln abgefüllt werden, oder auch Mehrschichttabletten oder -dragees, bei denen neben der Retardschicht in einer anderen Schicht nicht retardierte Wirkstoffe enthalten sein sollen.

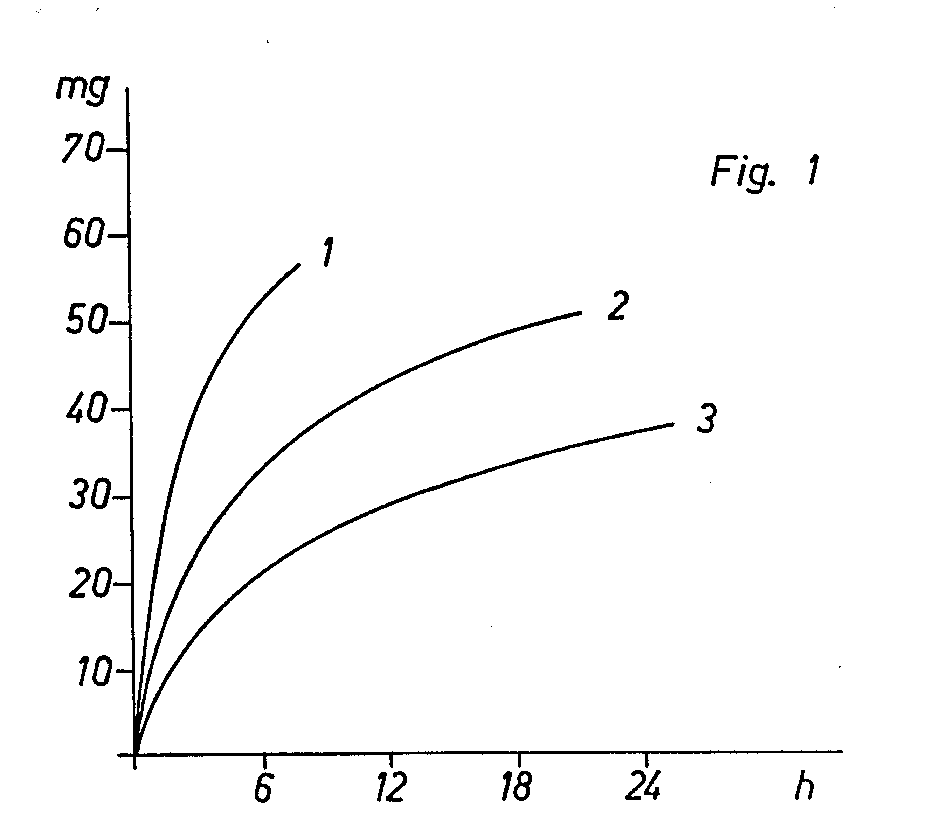

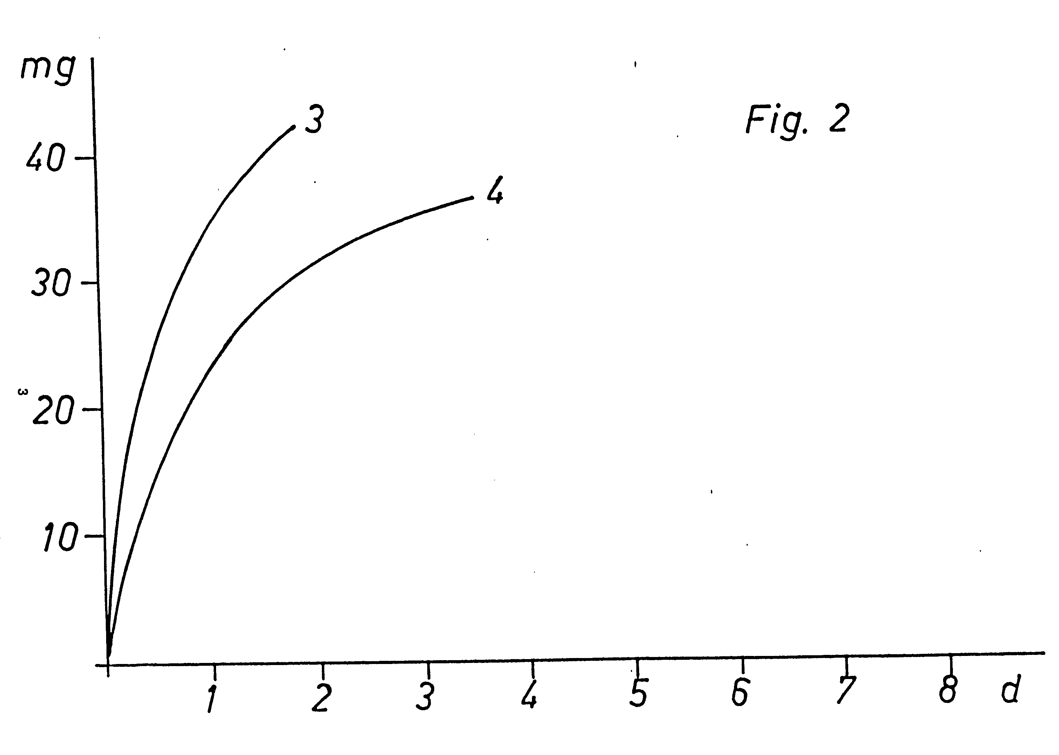

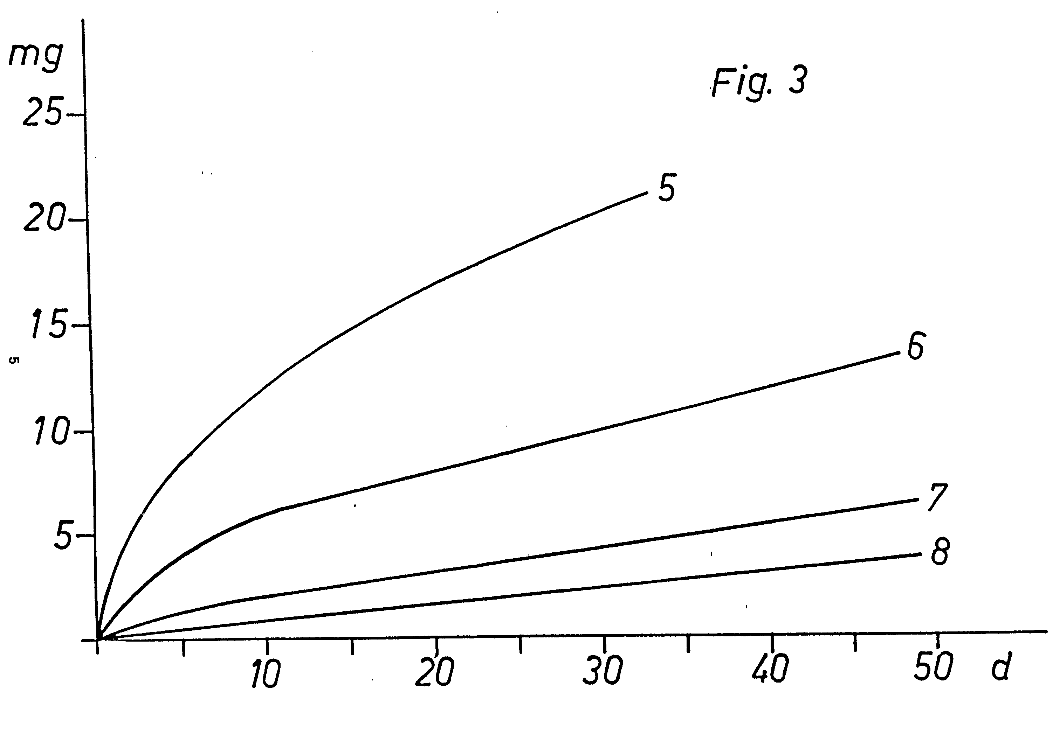

Die chemische Struktur der Poly-D(-)3-hydroxybuttersäure ist die eines linearen Polymeren, wobei 500 bis 25 000 Wiederholungseinheiten gefunden werden können. Für die Herstellung der erfindungsgemäßen Matrixformen sind Polymere mit einem Molekulargewicht von 25 000 bis 1 000 000 besonders geeignet. Die zur Herstellung der Preßlinge erforderliche Menge an Poly-D(-)-3-hydroxybuttersäure richtet sich nach der gewünschten Freisetzungsgeschwindigkeit des Wirkstoffes und beträgt zur Erzielung eines guten Retardeffektes mindestens 20 Gew.%, bezogen auf die Wirkstoffmenge, unterliegt aber sonst keiner Beschränkung. Der Retardeffekt ist in hohem Maß abhängig von der Menge der zugesetzten Poly-D(-)-3-hydroxybuttersäure, sodaß sich die Freisetzungsgeschwindigkeit des Wirkstoffes im Zeitraum von mehreren Stunden bis zu mehreren Monaten praktisch beliebig einstellen läßt (vgl. dazu Figur 1 bis 3).

Als Retardpräparate für die orale Applikation sind Matrixformen mit einem hohen Wirkstoffgehalt und einem geringen Anteil an Poly-D(-)-3-hydroxybuttersäure, der je nach Art des Wirkstoffes und der erwünschten Freisetzungsrate beispielsweise 20 bis 40 Gew.%, bezogen auf das Gesamtgewicht aus Wirkstoff und Poly-D(-)-3-hydroxybuttersäure, betragen kann, besonders geeignet. Für die parenterale Applikation, beispielsweise als Implantate, bei deren Anwendung der Wirkstoff nach der Einpflanzung im Körper zugleich mit dem biologischen Abbau des erfindungsgemäßen Trägermaterials im Zeitraum von einer Woche bis zu mehreren Monaten gleichmäßig freigesetzt werden soll, sind dagegen Matrixformen mit einem geringen Wirkstoffanteil und einem hohen Anteil an Poly-D(-)-3-hydroxybuttersäure, beispielsweise von 70 bis über 95 Gew.%, bezogen auf das Gesamtgewicht aus Wirkstoff und Poly-D(-)-3-hydroxybuttersäure, empfehlenswert.

Die für den erfindungsgemäßen Zweck benötigte Poly-D(-)-3-hydroxybuttersäure ist zum Beispiel nach dem biologischen Verfahren von Lafferty et al., Chem. Rundschau 30 (41) 14 bis 16, 1977, relativ einfach auch in großen Mengen erhältlich. Ein weiterer Gegenstand der Erfindung ist ein Verfahren zur Herstellung dieser Preßlinge mit retardierter Wirkstoff-Freisetzung.

Die Poly-D(-)-3-hydroxybuttersäure weist ganz im Gegensatz zu den Polylactiden einerseits geringe elastische Eigenschaften und geringe elektrostatische Aufladungstendenz auf und verfügt andererseits über gute Schmier- und Gleiteigenschaften. Der Wirkstoff kann daher auf einfache Weise mit dem erfindungsgemäßen Trägermaterial vermischt und in eine homogene Form gebracht werden. Das Homogenisieren kann bei geringen Mengen mittels Pulverreibschale und Pistill oder einer Pulvermischdose erfolgen. Große Ansätze lassen sich mit Hilfe von rotierenden Trommeln, Schaufelmischern, Tellermischern, Mischschnecken, Ribbonmischern, Konusmischern, Doppelkonusmischern und V-Mischern (Twin-Shellblender) homogenisieren.

Die auf diese Weise erhaltenen Homogenate können direkt ohne weitere Behandlung und ohne weitere Zuschlagsstoffe zu Tabletten, Drageekernen oder anderen Komprimaten beliebiger Form verpreßt werden. Zieht man die Schwierigkeiten in Betracht, die bei den bisher bekannten biologisch abbaubaren Matrixformen in der Arzneiformengebung auftreten, so ist es für einen Fachmann überraschend, daß man im erfindungsgemäßen Matrixsystem durch einfaches Mischen und Verpressen feste Arzneiformen mit nahezu beliebig variierbarer Freisetzungsrate herstellen kann.

Die erfindungsgemäß erhältlichen Homogenate aus dem Wirkstoff und der Poly-D(-)-3-hydroxybuttersäure können selbstverständlich vor dem Verpressen auch nach einem der üblichen Granulierverfahren zu einem Granulat verarbeitet werden oder, wenn gewünscht, mit den in der Galenik üblichen Hilfs- und Zuschlagsstoffen versetzt werden.

Das Herstellen der Preßlinge ist mit allen herkömmlichen Hand- oder Automatenpressen möglich, wobei wegen der guten Schmier- und Gleiteigenchaften der Poly-D(-)-3-hydroxybuttersäure ein Zusatz von Schmier- oder Gleitmitteln unterbleiben kann.

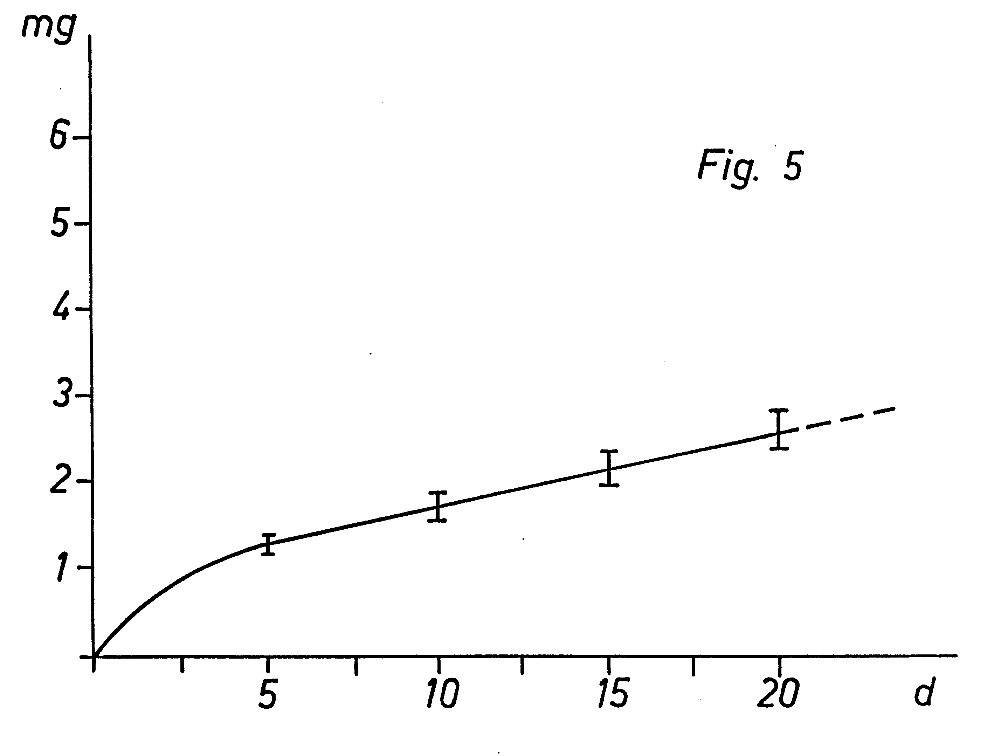

Der Preßdruck kann über den Bereich von 1 bis 20 Tonnen beliebig variiert werden. Die Freisetzungsrate des Wirkstoffes weist bei einer Variation des Preßdruckes im Bereich von 1 bis zu 10 Tonnen keine signifikante Abhängigkeit vom Preßdruck auf (vgl. dazu Figur 5).

Der biologische Abbau der Poly-D(-)-3-hydroxybuttersäure erfolgt im Körper entweder auf hydrolytischem oder auf enzymatischem Weg. Der Wirkstoff wird aus der erfindungsgemäßen Matrixform teils durch Oberflächenerosion, teils durch Diffusionsvorgänge über einen langen Zeitraum gleichmäßig und mit vollständigem biologischem Abbau der Matrix zur Gänze freigesetzt.

So konnte bei Untersuchungen über die Wirkstoff-Freisetzung in vivo, bei denen erfindungsgemäß hergestellte Preßlinge mit Theophyllin als Wirkstoff Mäusen subcutan in die Nackenfalte implantiert wurden, über 20 Wochen hinweg eine gleichmäßige Freisetzung des Wirkstoffes registriert werden.

Die Freisetzungsrate läßt sich durch das Verhältnis von Wirkstoffmenge zur Matrix (Beispiele 1 - 8, Figur 1 bis 3), durch die Tablettengröße (Beispiele 9 und 10, Figur 4) und in geringem Umfang durch den Preßdruck, bei dem die Preßlinge hergestellt werden, (Beispiel 11 Figur 5) in vielfältiger Weise beeinflussen. Diese Effekte lassen sich in sehr einfacher Weise an einem Freisetzungsmodell unter physiologischen Bedingungen in vitro darstellen.

In vitro Freisetzungsraten von Theophyllin:

In diesem Modellversuch werden zur in vitro-Untersuchung der Freisetzungsraten von Theophyllin die Tabletten entsprechend den physiologischen Bedingungen in 100 ml 0,9 %iger Natriumchloridlösung (NaCl:OAB) in 200 ml fassenden, verschlossenen Braunglasflaschen bei 37°C geschüttelt. Die analytische Auswertung erfolgte durch spektralphotometrische Messung bei 273 nm direkt bzw. in Verdünnung.

Anhand der folgenden Beispiele soll das erfindungsgemäße Verfahren erläutert werden.

**Beispiel 1:**

| Rezeptur: | 1 Stück | Ansatz für 30 000 Stück |
|---|---|---|
| 7-Hydroxyäthyltheophyllin | 56 mg | 1,68 kg |
| Poly-D(-)-3-hydroxybuttersäure | | |
| mit einem MG von ca. 100 000 | 14 mg | 0,42 kg |

Verarbeitung:
Die Bestandteile werden gesiebt, vermengt und in einem Konusmischer homogenisiert.
Das Homogenat wird bei einem Preßdruck von 10 Tonnen, entsprechend 981 N/mm$^2$ zu Tabletten verpreßt.

| Tablettengewicht: | 70 mg |
|---|---|
| Wirkstoffgehalt: | 80 Gew.% (56 mg) |
| Durchmesser: | 6,0 mm |
| Höhe: | 2,0 mm |
| Bruchfestigkeit: | 64 N (festgestellt mit Schleuninger Mod.2E/205) |

Unter den gleichen Verarbeitungsbedingungen, wie oben angegeben, wurden Tabletten mit folgendem Wirkstoffgehalt an 7-Hydroxy-äthyltheophyllin hergestellt:

**Beispiel 2:** Wirkstoffgehalt 70 Gew.% (49 mg) Matrix 30 Gew.%

**Beispiel 3:** Wirkstoffgehalt 60 Gew.% (42 mg) Matrix 40 Gew.%

**Beispiel 4:** Wirkstoffgehalt 50 Gew.% (35 mg) Matrix 50 Gew.%

**Beispiel 5:** Wirkstoffgehalt 30 Gew.% (21 mg) Matrix 70 Gew.%

**Beispiel 6:** Wirkstoffgehalt 20 Gew.% (14 mg) Matrix 80 Gew.%

**Beispiel 7:** Wirkstoffgehalt 10 Gew.% (7 mg) Matrix 90 Gew.%

**Beispiel 8:** Wirkstoffgehalt 5 Gew.% (3,5 mg) Matrix 95 Gew.%

Die Liberationskurven der Beispiele 1 bis 8 sind in den Figuren 1 bis 8 dargestellt, wobei sich die Nummern der Liberationskurven auf die der Beispiele 1 bis 8 beziehen.
Die Bruchfestigkeit der Tabletten erreicht einen Wert von 64 N bei einem Wirkstoffgehalt von 80 % bis 82 N bei einem Wirkstoffgehalt von 5 %.

**Beispiel 9:**

| Rezeptur: | 1 Stück | Ansatz für 10 000 Stück |
|---|---|---|
| 7-Hydroxyäthyltheophylllin | 7 mg | 70 g |
| Poly-D(-)-3-hydroxybuttersäure | 63 mg | 630 g |

Die Bestandteile werden wie in Beispiel 1 homogenisiert und das Homogenat bei einem Preßdruck von 10 Tonnen (981 N/mm$^2$) Tabletten verpreßt.

Tablettengewicht: 70 mg
Wirkstoffgehalt: 10 % (7 mg)
Durchmesser: 6,0 mm
Höhe: 2,0 mm

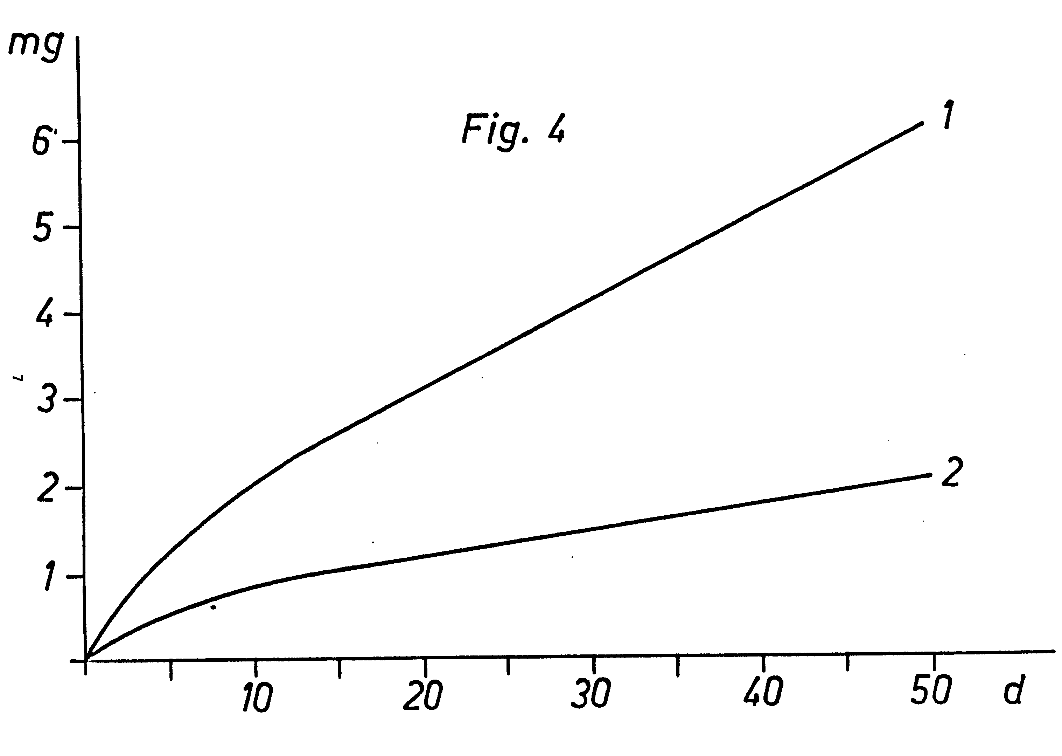

Die Liberationskurve ist in Figur 4, Nummer 1, dargestellt.

**Beispiel 10:**

| Rezeptur: | 1 Stück | Ansatz für 10 000 Stück |
|---|---|---|
| 7-Hydroxyäthyltheophyllin | 9 mg | 90 g |
| Poly-D(-)-3-hydroxybuttersäure | 81 mg | 810 g |

Die Bestandteile werden wie in Beispiel 1 homogenisiert und das Homogenat bei einem Preßdruck von 10 Tonnen (981 N/mm$^2$) zu Tabletten verpreßt.

Tablettengewicht: 90 mg
Wirkstoffgehalt: 10 % (9 mg)
Durchmesser: 8,0 mm
Höhe: 2,0 mm

Die Liberationskurve ist in Figur 4, Nummer 2, dargestellt.

**Beispiel 11:**

| Rezeptur: | 1 Stück | Ansatz für 10 000 Stück |
|---|---|---|
| 7-Hydroxyäthyltheophyllin | 7 mg | 70 g |
| Poly-D(-)-3-hydroxybuttersäure | 63 mg | 630 g |

Die Bestandteile werden wie in Beispiel 1 homogenisiert und das Homogenat wird bei einem Preßdruck von 2 Tonnen (Markierung oberhalb der Liberationskurve in Figur 5), 4, 6, 8 und 10 Tonnen (Markierung unterhalb der Liberationskurve in Figur 5) verpreßt. Die Freisetzungsraten zeigen wenig Abhängigkeit vom Preßdruck im Bereich von 2 bis 10 Tonnen (196-981 N/mm$^2$) und liegen auf der in Figur 5 gezeigten Liberationskurve innerhalb des durch die senkrechten Markierungen angegebenen Bereiches.

**Patentansprüche**

für die Vertragsstaaten BE, CH, DE, FR, GB, IT, LI, LU, NL, SE.

1. Preßlinge mit retardierter Wirkstofffreisetzung zur oralen und parenteralen Applikation von Arzneistoffen, bestehend aus dem Wirkstoff und einem polymeren Trägermaterial, dadurch gekennzeichnet, daß sie wenigstens einen pharmazeutisch aktiven Wirkstoff in homogener Mischung mit mindestens 20 Gew.% Poly-D(-)-3-hydroxybuttersäure, bezogen auf die Wirkstoffmenge, enthalten.

2. Preßlinge nach Anspruch 1, dadurch gekennzeichnet, daß die Poly-D(-)-3-hydroxybuttersäure ein Molekulargewicht von 25 000 bis 1 000 000 besitzt.

3. Preßlinge nach den Ansprüchen 1 und 2 zur oralen Langzeitapplikation von Arzneistoffen, dadurch gekennzeichnet, daß sie eine homogene Mischung des pharmazeutisch aktiven Wirkstoffs mit 20 bis 40 Gew.% Poly-D(-)-3-hydroxybuttersäure bezogen auf das Gesamtgewicht aus Wirkstoff und Poly-D(-)-3-hydroxybuttersäure enthalten.

4. Preßlinge nach den Ansprüchen 1 und 2 zur parenteralen Langzeitapplikation von Arzneistoffen, dadurch gekennzeichnet, daß sie eine homogene Mischung des pharmazeutisch aktiven Wirkstoffs mit 70 bis 95 Gew.% Poly-D(-)-3-hydroxybuttersäure bezogen auf das Gesamtgewicht aus Wirkstoff und Poly-D(-)-3-hydroxybuttersäure enthalten.

5. Preßlinge nach den Ansprüchen 1 bis 4, dadurch gekennzeichnet, daß die homogene Mischung in der Galenik übliche Hilfs- und Zuschlagstoffe enthält.

6. Verfahren zur Herstellung von Preßlingen nach den Ansprüchen 1 bis 5, dadurch gekennzeichnet, daß der Wirkstoff mit der Poly-D(-)-3-hydroxybuttersäure auf mechanische Weise vermischt und homogenisiert wird, worauf aus dem erhaltenen Homogenat unter einem Druck von 98,1-1962 N/mm$^2$ (1 bis 20 Tonnen) Preßlinge hergestellt werden.

7. Verfahren nach Anspruch 6, dadurch gekennzeichnet, daß die homogenisierte Mischung zu Tabletten verpreßt wird.

8. Verfahren nach Anspruch 6, dadurch gekennzeichnet, daß die homogenisierte Mischung zu Drageekernen verpreßt wird.

## Patentansprüche

für den Vertragsstaat AT:

1. Verfahren zur Herstellung von Preßlingen mit retardierter Wirkstofffreisetzung zur oralen oder parenteralen Applikation von Arzneistoffen, bestehend aus dem Wirkstoff und einem polymeren Trägermaterial, dadurch gekennzeichnet, daß der Wirkstoff mit mindestens 20 Gew.% Poly-D(-)-3-hydroxybuttersäure, bezogen auf die Wirkstoffmenge auf mechanische Weise vermischt und homogenisiert wird, worauf aus dem erhaltenen Homogenat unter einem Druck von 98,1-1962 N/mm$^2$ (1 - 20 Tonnen) Preßlinge hergestellt werden.

2. Verfahren nach Anspruch 1, dadurch gekennzeichnet, daß eine Poly-D(-)3-hydroxybuttersäure mit einem Molekulargewicht von 25 000 bis 1 000 000 eingesetzt wird.

3. Verfahren nach den Ansprüchen 1 und 2, dadurch gekennzeichnet, daß aus einem Homogenat mit einem Gehalt von 20 bis 40 Gew.% Poly-D(-)-3-hydroxybuttersäure, bezogen auf den Wirkstoffgehalt, Preßlinge für die orale Applikation hergestellt werden.

4. Verfahren nach den Ansprüchen 1 und 2, dadurch gekennzeichnet, daß aus einem Homogenat mit einem Gehalt von 70 bis 95 Gew.% Poly-D(-)-3-hydroxybuttersäure, bezogen auf den Wirkstoffgehalt, Preßlinge für die parenterale Implantation hergestellt werden.

5. Verfahren nach den Ansprüchen 1 bis 4, dadurch gekennzeichnet, daß dem Homogenat in der Galenik übliche Hilfs- und Zuschlagstoffe zugesetzt werden.

6. Verfahren nach den Ansprüchen 1 bis 5, dadurch gekennzeichnet, daß das Homogenat zu Tabletten verpreßt wird.

7. Verfahren nach den Ansprüchen 1 bis 5, dadurch gekennzeichnet, daß das Homogenat zu Drageekernen verpreßt wird.

## Claims

for the contracting state AT.

1. Process for the preparation of compressed articles with delayed release of active compound, for the oral or parenteral administration of medicaments, consisting of the active compound and of a polymeric vehicle, characterized in that the active compound is mechanically mixed and homogenized with at least 20 % by weight of poly-D(-)-3-hydroxybutyric acid based on the amount of active compound, whereupon compressed articles are prepared from the resulting homogenate under a pressure of 98.1-1962 N/mm$^2$ (1-20 tonnes).

2. Process according to Claim 1, characterized in that a poly-D(-)-3-hydroxybutyric acid having a molecular weight of 25,000 to 1,000,000 is used.

3. Process according to Claims 1 and 2, characterized in that compressed articles for oral administration are prepared from a homogenate containing 20 to 40 % by weight of poly-D(-)-3-hydroxybutyric acid based on the content of active compound.

4. Process according to Claims 1 and 2, characterized in that compressed articles for parenteral implantation are prepared from a homogenate containing 70 to 95 % by weight of poly-D(-)-3-hydroxybutyric acid based on the content of active compound.

5. Process according to Claims 1 to 4, characterized in that auxiliaries and additives customary in pharmacy are added to the homogenate.

6. Process according to Claims 1 to 5, characterized in that the homogenate is compressed to form tablets.

7. Process according to Claims 1 to 5, characterized in that the homogenate is compressed to form cores for coated tablets.

# 0 108 882

## Claims

for the contracting states BE, CH, DE, FR, GB, IT, LI, LU, NL, SE.

1. Compressed articles with delayed release of active compound, for the oral and parenteral administration of medicaments, consisting of the active compound and of a polymeric vehicle, characterized in that they contain at least one pharmaceutically active compound homogeneously mixed with at least 20 % by weight of poly-D(-)-3-hydroxybutyric acid based on the amount of active compound.

2. Compressed articles according to Claim 1, characterized in that the poly-D(-)-3-hydroxybutyric acid has a molecular weight of 25,000 to 1,000,000.

3. Compressed articles according to Claims 1 and 2 for long-term oral administration of medicaments, characterized in that they contain a homogeneous mixture of the pharmaceutically active compound with 20 to 40 % by weight of poly-D(-)-3-hydroxybutyric acid based on the total weight of active compound and poly-D(-)-3-hydroxybutyric acid.

4. Compressed articles according to Claims 1 and 2 for long-term parenteral administration of medicaments, characterized in that they contain a homogeneous mixture of the pharmaceutically active compound with 70 to 95 % by weight of poly-D(-)-3-hydroxybutyric acid based on the total weight of active compound and poly-D(-)-3-hydroxybutyric acid.

5. Compressed articles according to Claims 1 to 4, characterized in that the homogeneous mixture contains auxiliaries and additives customary in pharmacy.

6. Process for the preparation of compressed articles according to Claims 1 to 5, characterized in that the active compound is mechanically mixed and homogenized with the poly-D(-)-3-hydroxybutyric acid, whereupon compressed articles are prepared from the resulting homogenate under a pressure of 98.1-1962 N/mm (1 to 20 tonnes).

7. Process according to Claim 6, characterized in that the homogenized mixture is compressed to form tablets.

8. Process according to Claim 6, characterized in that the homogenized mixture is compressed to form cores for coated tablets.

## Revendications

pour les Etats contractants BE, CH, DE, FR, GB, IT, LI, LU, NL, SE.

1. Comprimés à libération retardée du produit actif pour application orale et parentérale de médicaments, comprenant le produit actif et une matière de support polymère, caractérisés en ce qu'ils renferment au moins un produit pharmaceutiquement actif en mélange homogène avec au moins 20 % en poids d'acide poly-D(-)-3-hydroxybutyrique, le pourcentage étant rapporté à la quantité de produit actif.

2. Comprimés suivant la revendication 1, caractérisés en ce que l'acide poly-D(-)-3-hydroxybutyrique présente un poids moléculaire de 25.000 à 1.000.000.

3. Comprimés suivant les revendications 1 et 2 pour application orale à long terme de médicaments, caractérisés en ce qu'ils renferment un mélange homogène du produit pharmaceutiquement actif avec 20 à 40 % en poids de poly-D(-)-3-hydroxybutyrique, le pourcentage étant rapporté au poids total de produit actif et d'acide poly-D(-)-3-hydroxybutyrique.

4. Comprimés suivant les revendications 1 et 2 pour application parentérale à long terme de médicaments, caractérisés en ce qu'ils renferment un mélange homogène du produit pharmaceutiquement actif avec 70 à 95 % en poids d'acide poly-D(-)-3-hydroxybutyrique, le pourcentage étant rapporté au poids total de produit actif et d'acide poly-D(-)-3-hydroxybutyrique.

5. Comprimés suivant les revendications 1 à 4, caractérisés en ce que le mélange homogène renferme des adjuvants et des additifs usuels en galénique.

6. Procédé de préparation de comprimés suivant les revendications 1 à 5, caractérisé en ce qu'on mélange le produit actif avec l'acide poly-D(-)-3-hydroxybutyrique de façon mécanique et l'on homogénéise le mélange, et l'on produit alors des comprimés à partir de l'homogénat obtenu sous une pression de 98,1 à 1,962 N/mm$^2$ (1 à 20 tonnes).

7. Procédé suivant la revendication 6, caractérisé en ce qu'on comprime le mélange homogénéisé en pastilles.

8. Procédé suivant la revendication 6, caractérisé en ce qu'on comprime le mélange homogénéisé en noyaux de dragées.

**Revendications**

pour l'Etat contractant AT.

1. Procédé de production de comprimés à libération retardée du produit actif pour application orale ou parentérale de médicaments, comprenant le produit actif et une matière de support polymère, caractérisé en ce qu'on mélange le produit actif avec un pourcentage d'au moins 20 % en poids d'acide poly-D(-)-3-hydroxybutyrique rapporté à la quantité de produit actif de façon mécanique et l'on homogénéise le mélange, et l'on produit alors des comprimés à partir de l'homogénat obtenu sous une pression de 98,1 à 1.962 N/mm² (1 à 20 tonnes).

2. Procédé suivant la revendication 1, caractérisé en ce qu'on utilise un acide poly-D(-)-3-hydroxybutyrique d'un poids moléculaire de 25.000 à 1.000.000.

3. Procédé suivant les revendications 1 et 2, caractérisé en ce qu'on produit des comprimés pour application orale à partir d'un homogénat d'une teneur de 20 à 40 % en poids d'acide poly-D(-)-3-hydroxybutyrique, le pourcentage étant rapporté à la teneur en produit actif.

4. Procédé suivant les revendications 1 et 2, caractérisé en ce qu'on produit des comprimés pour implantation parentérale à partir d'un homogénat d'une teneur de 70 à 95 % en poids d'acide poly-D(-)-3-hydroxybutyrique, le pourcentage étant rapporté à la teneur en produit actif.

5. Procédé suivant les revendications 1 à 4, caractérisé en ce qu'on ajoute à l'homogénat des adjuvants et additifs usuels en galénique.

6. Procédé suivant les revendications 1 à 5, caractérisé en ce qu'on comprime l'homogénat en pastilles.

7. Procédé suivant les revendications 1 à 5, caractérisé en ce qu'on comprime l'homogénat en noyaux de dragées.

Fig. 1

Fig. 2

Fig. 3

Fig. 4

0 108 882

Fig. 5

0 108 882